# EUROPÄISCHE PATENTSCHRIFT

(11) **EP 2 370 012 B1**
(45) Veröffentlichungstag und Bekanntmachung des Hinweises auf die Patenterteilung: **23.10.2013**
(21) Anmeldenummer: 09740136.8
(22) Anmeldetag: 23.10.2009
(51) Int. Cl.: A61B 17/80, A61B 17/86

(54) **IMPLANTAT UND KNOCHENSCHRAUBE MIT INEINANDERGREIFENDEN NOCKEN**
IMPLANT AND BONE SCREW HAVING INTERLOCKING CAMS
IMPLANT ET VIS À OS COMPRENANT DES ERGOTS ENTRANT EN PRISE

(30) Priorität: 31.10.2008 DE 102008043370
(43) Veröffentlichungstag der Anmeldung: 05.10.2011
(73) Patentinhaber: Universität Rostock, 18055 Rostock (DE)
(72) Erfinder: GRADL, Georg, 18211 Börgerende (DE)
(74) Vertreter: Wablat Lange Karthaus
(86) Internationale Anmeldenummer: PCT/EP2009/063981
(87) Internationale Veröffentlichungsnummer: WO 2010/049361

(56) Entgegenhaltungen:
- WO-A2-2007/014192
- DE-A1- 4 343 117
- US-A1- 2008 097 444
- US-A1- 2008 234 677

## Beschreibung

Die Erfindung bezieht sich auf eine Fixationsvorrichtung für Knochen, aus einem am Knochen zu befestigenden Implantat mit wenigstens einem Durchgangsloch und aus wenigstens einer im Durchgangsloch zu befestigenden Knochenschraube, wobei im Durchgangsloch und am oberen Ende der Knochenschraube miteinander in Eingriff stehende Befestigungselemente angeordnet sind.

Zur Behandlung von Frakturen vornehmlich an den gelenktragenden Enden der langen Röhrenknochen sind Implantate in Form von Platten aus Stahl oder Titan notwendig, die idealer Weise mit Knochenschrauben fixiert werden. Diese werden fest mit dem Implantat verankert. Dies erfolgt dadurch, dass die Knochenschraube und das Implantat ein einander komplementäres Gewinde haben. Es entsteht nach dem Eindrehen der Knochenschraube in das Implantat eine winkelstabile Fixationsvorrichtung, jedoch kann die Knochenschraube aus dem rechten Winkel bei einer Implantatentfernung meist nicht ohne erhebliche Kraftanstrengung gelöst werden.

Erreicht wird hierdurch eine größere Steifigkeit einer Osteosynthese, was durch eine geringe Rate an Implantatversagen positiv zur Geltung kommt. Ein Problem besteht jedoch darin, dass die Knochenschraube nur in einem bestimmten Winkel, nämlich dem Winkel des Gewindes, in das zugehörige Implantat eingebracht werden kann. Das setzt voraus, dass das Implantat optimal einem Knochen angepasst bzw. angebogen werden muss. Interindividuelle Unterschiede der Anatomie des Menschen und Unterschiede in der Fakturgeometrie müssen unberücksichtigt bleiben.

In jüngster Vergangenheit wurde die monoaxiale Winkelstabilität der herkömmlichen Implantate in eine polyaxiale Winkelstabilität umgewandelt, welche durch Schneiden eines Gewindes beim Eindrehen der Knochenschraube durch den Operateur erreicht wird. Hierbei wird für die Schraube eine harte Titanlegierung gewählt, für das Implantat und damit für das Durchgangsloch hingegen ein weiches Material bzw. eine weiche Legierung. Nachteilig hierbei ist der entstehende Materialabrieb.

In einer Variante nach DE 10 2005 015 496 A1 wird das Gewinde in verschiedenen Richtungen in das Durchgangsloch im Implantat geschnitten, so dass der Chirurg zwischen fünf verschiedenen Winkeln wählen kann. Dies erfordert jedoch einen erheblichen Aufwand bei der Herstellung des Implantates.

Bei monoaxial winkelstabilen Implantaten besteht beim Eindrehen der mit Gewinde versehenen Knochenschraube in das mit Gewinde versehene Durchgangsloch des Implantats die Gefahr, dass eine Kaltverschweißung dann erfolgt, wenn beim Eindrehen der Knochenschraube in einen Gewindegang des Implantates eine Verkippung auftritt und die Knochenschraube nicht mehr genau in das komplementäre Gewinde des Implantates passt. Hierbei ereignen sich Verformungen, die zu einem sehr festen Sitz der Knochenschraube in der Gewindebohrung führen. Dies ist ungewollt und macht das spätere Entfernen der Knochenschraube im Falle einer Implantatentfernung sehr schwierig. Auch alle Lösungen der polyaxialen Winkelstabilität führen, auch wenn dies konzeptionell nicht vorgesehen ist, zu einer Verformung der Gewindegänge, die jedoch nur punktuelle auftritt, da die Gewinde nicht komplett ausgebildet sind, sondern nur Gewindefragmente aufweisen.

Eine Fixationsvorrichtung ähnlicher Art ist aus DE 198 58 889 B1 vorbekannt. Hierbei sind die Befestigungselemente an der Knochenschraube als vorgeformtes Gewinde und im Durchgangsloch des Implantates als kreisringförmiger Vorsprung ausgebildet. Die Knochenschraube st in verschiedenen Winkelstellungen bezüglich der Achse des Durchgangsloches in das Implantat eindrehbar. Auch bei dieser Fixationsvorrichtung besteht die Gefahr der Kaltverschweißung zwischen Knochenschraube und Implantat.

DE 43 43 117 A1 offenbart eine Fixationsvorrichtung gemäß dem Oberbegriff des Anspruchs 1.

Der Erfindung liegt die Aufgabe zugrunde, eine Fixationsvorrichtung der gattungsgemäßen Art dahingehend auszubilden, dass verschiedene Winkelstellungen der Knochenschraube zum Implantat ohne Kaltverschweißung möglich sind.

Zur Lösung dieser Aufgabe sieht die Erfindung vor, dass als Befestigungselemente ineinander greifende Nocken auf den zugeordneten Oberflächen des Durchgangsloches und der Knochenschraube angeordnet sind. Das Eindrehen der mit Nocken versehenen Knochenschraube in die ebenfalls mit Nocken versehene Durchgangsbohrung des Implantates ist in verschiedenen Winkelstellungen der Knochenschraube zum Implantat möglich, d. h. sowohl monoaxial als auch polyaxial, ohne dass eine Kaltverschweißung zwischen den beiderseitigen Nocken erfolgt.

Außerdem sind die Nocken aus Gewindeelementen gebildet. Diese können durch einzelne Gewindegänge gebildet werden, die achsparallel durchtrennt und damit zu einzelnen Nocken ausgeformt werden.

Gemäß der Erfindung weist die Knochenschraube wenigstens zwei unterschiedliche, übereinandergeschnittene Gewinde auf, wobei die Achse des einen Gewindes koaxial zur Achse der Knochenschraube und die Achse des zweiten Gewindes in einem spitzen Winkel zur Achse der Knochenschraube verläuft. Diese Lösung, bei der auf eine Knochenschraube Gewinde in unterschiedlicher Richtung aufgebracht bzw. aufgeschnitten werden, führt zu einer Gewindefragmentierung, die sowohl eine Monoaxialität als auch eine Polyaxialität der Knochenschraube zum Implantat zulässt, und auch eine Kaltverschweißung vermeidet. Dies wird dadurch erreicht, dass auf die Knochenschraube in einem ersten Arbeitsgang das erste Gewinde aufgeschnitten wird und dann der Gewindeschneider gekippt wird, um das zweite Gewinde in einem anderen, spitzen Winkel zur Achse der Knochenschraube zu formen. Dies führt zu einer Gewindefragmentierung. Die entstehenden Nocken aus den Gewinderesten führen beim Zusammenfügen von Knochenschraube und Implantat nicht zu einer Kaltverformung. Hierbei werden multiplen Gewindegänge auf die Knochenschraube aufgebracht. Das Durchgangloch im Implantat bedarf keines polyaxialen Gewindes.

In noch weiterer vorteilhafter Ausgestaltung der Erfindung ist im Durchgangsloch des Implantates wenigstens ein Gewindegang vorgesehen, wobei zur Erreichung der notwendigen Stabilität zweckmäßiger Weise zwei Gewindegänge vorgesehen werden, die zu Gewindefragmenten ausgebildet werden. Der zweite Gewindegang kann auch als Ring ohne Steigung ausgebildet sein.

In noch weiterer Ausgestaltung der Erfindung ist das Durchgangsloch im Implantat auf beiden Seiten des Implantates konisch ausgeformt, um die polyaxiale Befestigung der Knochenschraube im Implantat unter verschiedenen spitzen Winkeln zu ermöglichen.

Die Erfindung ist nachfolgend anhand von zwei in den Zeichnungen dargestellten Fixationsvorrichtungen für Knochen aus einem Implantat und einer Knochenschraube näher erläutert. Es zeigt:
- Fig. 1: eine Ansicht auf eine Knochenschraube,
- Fig. 2: eine Teilansicht auf das Implantat für die Knochenschraube nach Fig. 1,
- Fig. 3: eine Ansicht auf die Knochenschraube in einer Ausführungsform der Erfindung und
- Fig.4: einen Schnitt durch das Durchgangsloch im Implantat für die Knochenschraube nach Fig. 3.

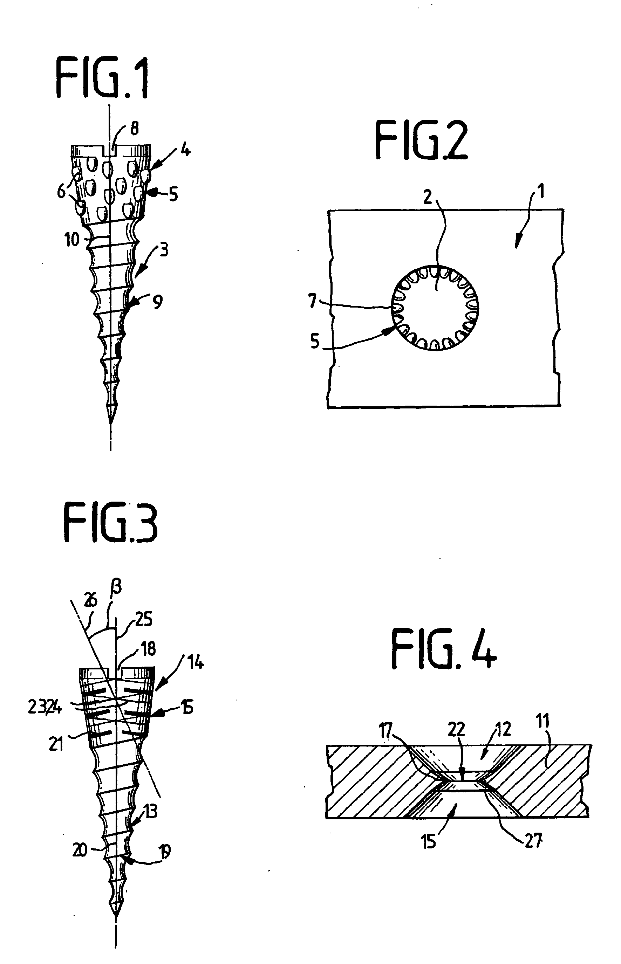

Eine Fixationsvorrichtung für Knochen besteht gemäß Fig. 1 und 2 aus einem am Knochen zu befestigenden Implantat 1 aus weichem metallischen Material mit wenigstens einem Durchgangsloch 2 und aus wenigstens einer im Durchgangsloch 2 zu befestigenden Knochenschraube 3, vorzugsweise aus Titan bzw. einer Titanlegierung. Im Durchgangsloch 2 und am vom Kopf 4 gebildeten oberen Ende der Knochenschraube 3 sind miteinander in Eingriff stehende Befestigungselemente 5 angeordnet. Diese sind aus Nocken 7 auf den zugeordneten Oberflächen des Durchgangsloches 2 und aus Nocken 6 auf der Knochenschraube 3 ausgebildet.

Wie es Fig. 1 zeigt, ist der Kopf 4 am oberen Ende der Knochenschraube 3 konisch ausgebildet und mit einem Schlitz 8 zum Eingriff eines Werkzeuges versehen. Der Schaft 9 ist in Richtung der Achse 10 mit einem speziellen Einschraubgewinde für den Knochen versehen.

Das Durchgangsloch 2 im Implantat 1 kann auf beiden Seiten des Implantates 1 konisch ausgeformt sein.

Bei der in den Fig. 3 und 4 dargestellten Ausführungsform der Erfindung sind die als Befestigungselemente 15 vorgesehenen Nocken 16, 17 aus Gewindeelementen 21, 22 gebildet. Die Knochenschraube 13 weist am konischen Kopf 14 wenigstens zwei Gewinde 23, 24 auf, wobei die Achse 25 des einen Gewindes 23 koaxial zur Achse 20 der Knochenschraube 13 und die Achse 26 des zweiten Gewindes 24 in einem spitzen Winkel β zur Achse 20 der Knochenschraube 13 verläuft. Durch die unterschiedlichen, auf- bzw. übereinander geschnittenen Gewinde 23, 24 werden einzelne Teile der Gewindegänge abgearbeitet, so dass die Gewindeelemente 21, 22 in Form von Nocken 16, 17 als Befestigungselemente 15 stehen bleiben.

Im Durchgangsloch 12 des Implantates 11 ist wenigstens ein Gewindegang 27 vorgesehen. Ferner ist das Durchgangsloch 12 des Implantates 11 auf beiden Seiten des Implantates 11 konisch ausgeformt ist.

### Bezugszeichenliste

- 01: Implantat
- 02: Durchgangsloch
- 03: Knochenschraube
- 04: Kopf
- 05: Befestigungselement
- 06: Nocken
- 07: Nocken
- 08: Schlitz
- 09: Schaft
- 10: Achse
- 11: Implantat
- 12: Durchgangsloch
- 13: Knochenschraube
- 14: Kopf
- 15: Befestigungselement
- 16: Nocken
- 17: Nocken
- 18: Schlitz
- 19: Schaft
- 20: Achse
- 21: Gewindeelement
- 22: Gewindeelement
- 23: Gewinde
- 24: Gewinde
- 25: Achse
- 26: Achse
- 27: Gewindegang

## Patentansprüche

1. Fixationsvorrichtung für Knochen, aus einem an den Knochen zu befestigenden Implantat mit wenigstens einem Durchgangsloch und aus wenigstens einer im Durchgangsloch zu befestigenden Knochenschraube, wobei im Durchgangsloch des Implantates und am oberen Ende der Knochenschraube miteinander in Eingriff stehende Befestigungselemente angeordnet sind, wobei
als Befestigungselemente (5; 15) ineinandergreifende Nocken (6, 7; 16, 17) auf den zugeordneten Oberflächen des Durchgangsloches (2; 12) und der Knochenschraube (3; 13) angeordnet sind, wobei
die Nocken (16, 17) aus Gewindeelementen (21, 22) gebildet sind,
**dadurch gekennzeichnet,**
**dass** die Knochenschraube (13) wenigstens zwei unterschiedliche, übereipfindergeschnittene Gewinde (23, 24) aufweist, so dass die Gewindeelemente (21, 22) in Form von Nocken (16, 17) als Befestigungselemente (15) gebildet sind,
wobei die Achse (25) des einen Gewindes (23) koaxial zur Achse (20) der Knochenschraube (13) und die Achse (26) des anderen Gewindes (24) in einem spitzen Winkel (β) zur Achse (20) der Knochenschraube (13) verlaufen.

2. Fixationsvorrichtung nach Anspruch 1, **dadurch gekennzeichnet, dass** ein Gewindegang (27) im Durchgangsloch (12) des Implantates (11) vorgesehen und das Durchgangsloch (12) des Implantates (11) auf beiden Seiten des einen Gewindeganges (27) des Implantates (11) konisch ausgeformt sind.

## Claims

1. A fixation device for bones, consisting of an implant to be fastened to the bone, having at least one through hole, and of at least one bone screw to be fastened in the through hole, wherein fastening elements, which are in engagement with one another, are arranged in the through hole of the implant and at the upper end of the bone screw, wherein
cams (6, 7; 16, 17) engaging into one another are arranged as fastening elements (5; 15) on the associated surfaces of the through hole (2; 12) and the bone screw (3; 13), wherein
the cams (16, 17) are formed from threaded elements (21, 22),
**characterized in that**
the bone screw (13) has at least two different threads (23, 24) cut over one another, so that the threaded elements (21, 22) are formed in the form of cams (16, 17) as fastening elements (15), wherein the axis (25) of the one thread (23) runs coaxially to the axis (20) of the bone screw (13) and the axis (26) of the other thread (24) runs at an acute angle (β) to the axis (20) of the bone screw (13).

2. The fixation device according to Claim 1, **characterized in that** a thread pitch (27) is provided in the through hole (12) of the implant (11), and the through hole (12) of the implant (11) is shaped conically on both sides of the one thread pitch (27) of the implant (11).

## Revendications

1. Dispositif de fixation pour os, composé d'au moins un implant se fixant à l'os et doté d'au moins un trou traversant et d'au moins une vis à os se fixant dans le trou traversant, dans lequel, dans le trou traversant de l'implant et à l'extrémité supérieure de la vis à os, des éléments de fixation en prise mutuelle sont disposés,
des cames (6, 7 ; 16, 17) en prise mutuelle faisant office d'éléments de fixation (5 ; 15) étant disposées sur les surfaces associées du trou traversant (2 ; 12) et de la vis à os (3 ; 13),
les cames (16, 17) étant constituées d'éléments filetés (21, 22),
**caractérisé en ce que**
la vis à os (13) présente au moins deux filetages (23, 24) différents à coupe superposée, de sorte que les éléments filetés (21, 22) sont constitués en tant qu'éléments de fixation (15) sous forme de cames (16, 17),
l'axe (25) d'un filetage (23) s'étendant de manière coaxiale à l'axe (20) de la vis à os (13) et l'axe (26) de l'autre filetage (24) suivant un angle aigu (β) par rapport à l'axe (20) de la vis à os (13).

2. Dispositif de fixation selon la revendication 1, **caractérisé en ce qu'**un pas de vis (27) est prévu dans le trou traversant (12) de l'implant (11) et que le trou traversant (12) de l'implant (11) a une conformation conique des deux côtés d'un pas de vis (27) de l'implant (11).
